Europäisches Patentamt

European Patent Office

Office européen des brevets

(11) Publication number: **0 257 928**
**A2**

# (12) EUROPEAN PATENT APPLICATION

(21) Application number: **87307176.5**

(22) Date of filing: **13.08.87**

(51) Int. Cl.⁴: **A61K 7/42**

(30) Priority: **22.08.86 US 899493**

(43) Date of publication of application:
**02.03.88 Bulletin 88/09**

(84) Designated Contracting States:
**DE FR GB IT**

(71) Applicant: **Kaidbey, Kays**
**2220 1/2 Lombard Street**
**Philadelphia, PA 19146(US)**

(72) Inventor: **Kaidbey, Kays**
**2220 1/2 Lombard Street**
**Philadelphia, PA 19146(US)**

(74) Representative: **Goldin, Douglas Michael et al**
**J.A. KEMP & CO. 14, South Square Gray's Inn**
**London WC1R 5EU(GB)**

(54) **Suntanning composition.**

(57) A suntanning composition for topical application to skin to enhance melanogenesis (tanning) caused by ultraviolet-A wavelengths, said composition comprising a carrier vehicle and at least one effective essential oil in an amount which promotes said melanogenesis in the prsence of ultraviolet-A wavelengths.

EP 0 257 928 A2

## SUNTANNING COMPOSITION

### Background of the Invention

This invention relates generally to a suntanning composition, and in particular to a composition for topical application comprising at least one effective aromatic essential oil in a quantity which promotes melanogenesis (tanning).

The acquisition of a suntan is an activity pursued by many individuals in an effort to cosmetically improve their respective self-perceived appearances. This sunbathing occurs despite the deleterious effects that sunlight can have on a person's skin. In particular, the ultraviolet-B (UV-B) spectrum (290-320 nm) of sunlight is known to contribute to wrinkling, skin atrophy, elastosis (photoaging), and skin cancer. The sun's longer UV wavelengths (320-400 nm), known as ultraviolet-A (UV-A), also produce tanning, but are not associated with the magnitude of the above-recited UV-B deleterious effects. Thus, an individual can withstand a higher dosage of UV-A wavelengths.

While UV-A induced melanogenesis or pigmentation is a safer way than that presented by UV-B wavelengths for acquiring a suntan, UV-A wavelengths require long exposures for suntan production. UV-A induced suntans, however, are longer-lasting and generally are described as more attractive than those produced by UV-B wavelengths. Presently, for a sunbather to achieve a UV-A induced tan from natural sunlight, he must use a UV-B sunscreen composition, and remain in the sun for a relatively long period of time. If the sunbather uses no such sunscreen, he will acquire a faster tan because of the presence of the UV-B wavelengths, but he also risks a sunburn (phototoxic reaction) as well as the deleterious effects outlined above.

It is therefore evident that a need is present for a suntanning composition which enhances UV-A tanning. Accordingly, it is a primary object of the instant invention to provide a topically-applied suntanning composition which permits a sunbather to acquire a deeper tan in less time than presently required without such enhancement.

Another object of the invention is to provide a suntanning composition which promotes UV-A tanning and UV-B screening.

Yet another object of the invention is to provide a suntanning composition effective for both UV-A wavelengths as well as full-spectrum solar radiation.

These and other objects will become apparent throughout the description of the invention which follows.

### Summary of the Invention

The subject of the instant invention is a suntanning composition for topical application to skin to enhance melanogenesis (tanning) caused by ultraviolet-A (UV-A) wavelengths. The composition comprises a carrier vehicle and at least one effective essential oil in an amount which promotes melanogenesis in the presence of UV-A wavelengths. The composition can be used in the presence of natural full spectrum sunlight, a simulated full sunlight spectrum, or a UV-A wavelength spectrum only. In addition to at least one such essential oil, the composition can also comprise a sun-screen which acts to screen ultraviolet-B (UV-B) wavelengths from the user's skin. The carrier vehicle is employed to facilitate application of the composition to the skin.

Also a subject of the instant invention is a method of acquiring a suntan in the presence of UV-A wavelengths by the topical application of said suntanning composition to skin and the exposure of said skin so treated to UV-A wavelengths for a time period which is adequate to produce a suntan.

### Detailed Description of Preferred Embodiments

The subject of the instant invention is a suntanning composition that comprises at least one effective essential oil that acts to enhance tanning or melanogenesis of skin caused by ultraviolet-A (UV-A) wavelengths (320-400 nm) which constitute a part of the sun's spectrum. Essential oils are defined in this application to include naturally occurring essential oils as well as citrus oils.

One property common to the essential oils which are capable of enhancing UV-induced melanogenesis, in accordance with the present invention, is their potential to induce phototoxicity in human skin. However, not all essential oils are necessarily effective in enhancing tanning. It has been found that the source (country of origin) or the type of citrus or other essential oil greatly influences the degree of their potential to induce phototoxicity. This results in variations in their effectiveness in enhancing tanning. For example, as shown in Example 1 which follows, Lemon Oils from California had no activity in enhancing UVA-induced tanning. On the other hand, Oil Lemon from Italy was weakly active in enhancing melanogenesis.

In view of the above, it can be seen that the common denominator present in those essential oils which are effective in enhancing tanning in accordance with the present invention is their potential to produce phototoxicity in human skin. As a result, all essential oils, including naturally occurring essential oils as well as citrus oils, which possess this characteristic are deemed to be within the scope and contemplation of the present invention.

Heretofore, such phototoxic potential of essential oil has been considered by the cosmetic and fragrance industry to be a major undesirable side effect of these substances. Considerable effort has been expended in attempts to identify those oils which have such phototoxic potential so that they can be modified to eliminate the phototoxic potential or their use avoided altogether. Elimination of the phototoxic potential has been achieved predominately by subjecting these raw materials to various chemical processes such as distillation, etc., which appears to eliminate many of the photo-active constituents of the oils.

The above is to be contrasted with the present invention wherein the essential oils are specifically selected for their phototoxic potential, which potential is utilized to an advantage to enhance UVA-induced tanning. In accordance with the present invention, actual phototoxicity is totally prevented by the use of appropriate concentrations of the oil such that activity becomes limited to the enhancement of melanogenesis. In an alternate embodiment of the present invention, a sunscreen agent in incorporated in addition to the appropriate concentration of the oil, in order to permit interaction with appropriate UV wavelengths and to further block phototoxicity. Therefore, in accordance with the present invention, the potentially phototoxic essential oils, heretofore purposely avoided, can now be used to advantage for promoting UVA-induced tanning.


## EXAMPLE I

To demonstrate and identify certain essential oils, which are effective in enhancing melanogenesis (tanning), the following oils were obtained from Ungerer Company, Lincoln Park, New Jersey 07035: (1) Oil Lemon Star Brand FCC; (2) Lemon Oil Expressed; (3) Oil Orange Bitter FCC; (4) Oil Lime Expressed; (5) Oil Rue; and (6) Oil Orange FLA Mid Season FCC.

Each oil was diluted to the desired strength in an inert carrier vehicle of light mineral oil, and applied to 5 cm x 10 cm rectangular areas of the mid-backs of groups of five subjects who could readily tan; that is, subjects having skin types III-IV as defined in Federal Register 43:38280, 1978. The application dose was 5uL/cm², and was rubbed in gently and as uniformly as possible. Exposure to solar simulating radiation (SSR) and to UV-A wavelengths was given 90 minutes later to the test sites, during which time the subjects sat in the laboratory without activity. The UV-A wavelength dose was constant at 30J/cm², and is approximately equivalent to about two hours of UV-A wavelength in natural noon sunlight in July on a clear, cloudless day at the latitude of Philadelphia, PA. The minimal erythema dose with SSR was obtained a day previously by exposing skin sites of the subjects to 25% incremental doses of SSR from a xenon arc solar simulator source. This source is equipped with a dichroic mirror and a Schott WG 320 filter (1 mm thick) to produce solar simulation. A 1 mm UG 5 filter is also added to remove most of the residual infra-red heat wavelengths. UV-A wavelengths were obtained from this same source by using a 1mm thick Schoot WG 345 filter. The field size at skin level was approximately a 1.5 cm diameter circle and the mean intensity (flux) was about 70mw/cm² of SSR and 52.2mW/cm² of UV-A wavelengths.

Each subject also received UV-A exposure to a symmetric control area treated as described above with the carrier vehicle minus the essential oil. The UV-A wavelength dose was constant at 30J/cm2. At the end of irradiation, all sites were washed with soap and water, and dried. The results of these tests are shown in Table I, with the symbol "0" indicating no reaction; the symbol "+" indicating some reaction; and the symbol "++" indicating greater reaction. Concentrations are shown as weight percent of the entire composition. The grading is performed by visual judgment.

In all cases, the control areas showed no reaction. Enhanced tanning was evident with Oil Lime Expressed at 0.1 weight percent and 0.06 weight percent; with Oil Rue at 0.1 weight pecent and 0.06 weight percent; and with a combination of 0.04 weight percent Oil Lime Expressed and 0.02 weight percent Oil Rue. There was substantially no reaction with Lemon Oil expressed at 0.1 weight percent; Oil Lemon Star Brand FCC at 0.1 weight pecent; Oil Orange Bitter FCC at 0.1 weight percent; or Oil Orange FLA FCC at 0.1 weight pecent.

## TABLE I

### THE EFFECTIVENESS OF VARIOUS ESSENTIAL OILS IN ENHANCING UVA-INDUCED PIGMENTATION.  CONCENTRATIONS (%) IN LIGHT MINERAL OIL

| ESSENTIAL AROMATIC OIL | ACTIVITY (Enhancement of UVA-tanning) |
|---|---|
| Lemon Oil Expressed 0.1% (California) | 0 |
| Oil Lemon Star Brand FCC 0.1% (California) pressed | 0 |
| Oil Orange Bitter FCC 0.1% (California) | 0 |
| Oil Lemon Italian (Italy) 0.06% | 0 |
| Oil Lemon Italian (Italy) 0.1% | + |
| Oil Lime Expressed 0.1% | ++ |
| Oil Lime Expressed 0.06% | + |
| Oil Rue   0.1% | + |
| Oil Rue   0.06% | + |
| Oil Orange FLA FCC 0.1% (Florida) | 0 |
| Oil Lime Expressed 0.04% + Oil Rue   0.02% | + |

## EXAMPLE II

In the same manner as described in Example I, 10 Caucasian subjects of skin types III and IV, as defined in Federal Register 43:8280, 1978, had applied to their respective skin test site surfaces two areas of a composition containing 0.04 weight percent Oil Lime Expressed (L. Oil) and 0.02 weight percent Oil Rue (R. Oil) in light mineral oil; two areas of a composition containing 0.06 weight percent Oil Lime Expressed (L. Oil) in light mineral oil; and two areas of mineral oil only (Control). One and one-half to two hours later, the test sites were exposed to UV-A wavelengths from the filtered xenon solar simulator with the Schott WG 345 filter in place. Two dose levels of 30J/cm$^2$ and 40J/cm$^2$ were applied. The results are shown in Tables II and III, where visually determined grading is as follows: 0 = no result; 1 = minimal perceptible change; 2 = clear change with obvious borders; 3 = intense change; and 4 = deep, very intense change. (This grading method appears in all further Tables).

4

No erythema reactions were recorded at any time following irradiation. Intense pigmentation developed at all irradiated sites at the end of exposure which represented immediate pigment darkening. Pigmentation was recorded 10-14 days after exposure, with the test sites of all subjects except S.L. and O.K. showing a clear enhancement of pigmentation at both 30J and 40J/cm² dose levels.

**TABLE II**

UVA EXPOSURES TO TEST SITES TREATED WITH "ACTIVE" PRODUCTS AND WITH VEHICLE (CONTROL)

VIZ. MINERAL OIL UVA DOSE: 30J/cm²

| Subject Initials: | Age: | Sex: | Skin Type: | Test Site: | Erythema (hours) 24 Hours | 48 Hours | Pigmentation (10-14 Days) |
|---|---|---|---|---|---|---|---|
| J.W. | 18 | M | III | 0.04% L. Oil + 0.02% Rue Oil | 0 | 0 | 2 |
| | | | | 0.06% L. Oil | 0 | 0 | 3 |
| | | | | Control (Vehicle) | 0 | 0 | 1 |
| D.S | 25 | M | III | 0.04% L. Oil + 0.02% R. Oil | 0 | 0 | 2 |
| | | | | 0.06% L. Oil | 0 | 0 | 2 |
| | | | | Control | 0 | 0 | 1 |
| M.P. | 20 | M | IV | 0.04% L. Oil +0.02% R. Oil | 0 | 0 | 2 |
| | | | | 0.06% L. Oil | 0 | 0 | 2 |
| | | | | Control | 0 | 0 | 1 |
| R.M | 35 | M | III | 0.04% L. Oil + 0.02% R. Oil | 0 | – | 3 |
| | | | | 0.06% L. Oil | 0 | – | 1 |
| | | | | Control | 0 | – | 3 |
| S.L. | 21 | F | III | 0.04% L. Oil + 0.02% R. Oil | 0 | 0 | 2 |
| | | | | 0.06% L. Oil | 0 | 0 | 2 |
| | | | | Control | 0 | 0 | 1 |
| V.M. | 22 | M | III | 0.04% L. Oil + 0.02% R. Oil | 0 | 0 | 2 |
| | | | | 0.06% L. Oil | 0 | 0 | 2 |
| | | | | Control | 0 | 0 | 1 |
| E.H. | 23 | M | III | 0.04% L. Oil + 0.02% R. Oil | 0 | – | 3 |
| | | | | 0.06% L. Oil | 0 | – | 2 |
| | | | | Control | 0 | – | 1 |
| K.B. | 46 | F | III | 0.04% L. Oil + 0.02% R. Oil | 0 | 0 | 2 |
| | | | | 0.06% L. Oil | 0 | 0 | 2 |
| | | | | Control | 0 | 0 | 1 |
| O.K. | 29 | M | III | 0.04% L. Oil + 0.02% R. Oil | 0 | – | 2 |
| | | | | 0.06% L. Oil | 0 | – | 2 |
| | | | | Control | 0 | – | 2 |
| H.B. | 34 | M | III | 0.04% L. Oil + 0.02% R. Oil | 0 | 0 | 3 |
| | | | | 0.06% L. Oil | 0 | 0 | 3 |
| | | | | Control | 0 | 0 | 1 |

TABLE III
UVA EXPOSURES TO TEST SITES TREATED WITH "ACTIVE" PRODUCTS AND WITH VEHICLE (CONTROL)
VIZ. MINERAL OIL UVA DOSE: 40J/cm$^2$

| Subject Initials: | Age: | Sex: | Skin Type: | Test Site: | Erythema (hours) 24 Hours | 48 Hours | Pigmentation (10-14 Days) |
|---|---|---|---|---|---|---|---|
| J.W. | 18 | M | III | 0.04% L. Oil + 0.02% R. Oil | 0 | 0 | 3 |
| | | | | 0.06% L. Oil | 0 | 0 | 3 |
| | | | | Control (Vehicle) | 0 | 0 | 2 |
| D.S | 25 | M | III | 0.04% L. Oil + 0.02% R. Oil | 0 | 0 | 2 |
| | | | | 0.06% L. Oil | 0 | 0 | 3 |
| | | | | Control | 0 | 0 | 2 |
| M.P. | 20 | M | IV | 0.04% L. Oil + 0.02% R. Oil | 0 | 0 | 3 |
| | | | | 0.06% L. Oil | 0 | 0 | 3 |
| | | | | Control | 0 | 0 | 2 |
| R.M. | 35 | M | III | 0.04% L. Oil + 0.02% R. Oil | 0 | - | 3 |
| | | | | 0.06% L. Oil | 0 | - | 3 |
| | | | | Control | 0 | - | 2 |
| S.L. | 21 | F | III | 0.04% L. Oil + 0.02% R. Oil | 0 | 0 | 3 |
| | | | | 0.06% L. Oil | 0 | 0 | 3 |
| | | | | Control | 0 | 0 | 3 |
| V.M. | 22 | M | III | 0.04% L. Oil + 0.02 R. Oil | 0 | 0 | 2 |
| | | | | 0.06% L. Oil | 0 | 0 | 2 |
| | | | | Control | 0 | 0 | 1 |
| E.H. | 23 | M | III | 0.04% L. Oil + 0.02% R. Oil | 0 | - | 3 |
| | | | | 0.06% L. Oil | 0 | - | 3 |
| | | | | Control | 0 | - | 2 |
| K.B. | 46 | F | III | 0.04% L. Oil + 0.02% R. Oil | 0 | 0 | 2 |
| | | | | 0.06% L. Oil | 0 | 0 | 2 |
| | | | | Control | 0 | 0 | 1 |
| O.K. | 29 | M | III | 0.04% L. Oil + 0.02% R. Oil | 0 | - | 3 |
| | | | | 0.06% L. Oil | 0 | - | 3 |
| | | | | Control | 0 | - | 3 |
| H.B. | 34 | M | III | 0.04% L. Oil + 0.02% R. Oil | 0 | 0 | 3 |
| | | | | 0.06% L. Oil | 0 | 0 | 3 |
| | | | | Control | 0 | 0 | 2 |

## EXAMPLE III

Using the same composition formulations as in Example II, but with the addition of 3 weight percent UV-B wavelength sunscreen (Escalol 507, Van Dyk Company, Belleville, NJ; containing octyl dimenthyl PABA), each subject's skin had test sites prepared as shown in Table IV. With the exception of two subjects (R.G. and O.K.), a clear enhancement of pigmentation at a UV-A wavelength dose of 30J/cm$^2$ occurred.

## TABLE IV

**UVA EXPOSURES TO TEST SITES TREATED WITH "ACTIVE" PRODUCTS CONTAINING A SUNSCREEN (Escalol 507 at 3%) AND VEHICLE (CONTROL) VIZ. MINERAL OIL + A SUNSCREEN (507, 3%)**

| Subject Initials: | Age: | Sex: | Skin Type: | Test Site: | Erythema (hours) 24 Hours | 48 Hours | Pigmentation (10-14 Days) |
|---|---|---|---|---|---|---|---|
| J.W. | 18 | M | III | 0.04% L. Oil + 0.02% R. Oil +507 | 0 | 0 | 2 |
| | | | | 0.06% L. Oil + 507 | 0 | 0 | 2 |
| | | | | Control (Vehicle) + 507 | 0 | 0 | 1 |
| D.S. | 25 | M | III | 0.04% L. Oil + 0.02% R. Oil +507 | 0 | 0 | 2 |
| | | | | 0.06% L. Oil + 507 | 0 | 0 | 2 |
| | | | | Control + 507 | 0 | 0 | 1 |
| M.P. | 20 | M | IV | 0.04% L. Oil + 0.02% R. Oil +507 | 0 | 0 | 2 |
| | | | | 0.06% L. Oil + 507 | 0 | 0 | 2 |
| | | | | Control + 507 | 0 | 0 | 1 |
| R.M. | 35 | M | III | 0.04% L. Oil + 0.02% R. Oil +507 | 0 | 0 | 2 |
| | | | | 0.06% L. Oil + 507 | 0 | 0 | 2 |
| | | | | Control + 507 | 0 | 0 | 1 |
| R.G. | 33 | M | III | 0.04% L. Oil + 0.02% R. Oil +507 | 0 | 0 | 1 |
| | | | | 0.06% L. Oil + 507 | 0 | 0 | 1 |
| | | | | Control + 507 | 0 | 0 | 1 |

TABLE IV (continued)

**UVA EXPOSURES TO TEST SITES TREATED WITH "ACTIVE" PRODUCTS CONTAINING A SUNSCREEN (Escalol 507 at 3%)**
**AND VEHICLE (CONTROL) VIZ. MINERAL OIL + A SUNSCREEN (507, 3%)**

| Subject Initials: | Age: | Sex: | Skin Type: | Test Site: | Erythema (hours) 24 Hours | 48 Hours | Pigmentation (10-14 Days) |
|---|---|---|---|---|---|---|---|
| V.M. | 22 | M | III | 0.04% L. Oil + 0.02% R. Oil + 507 | 0 | 0 | 2 |
|  |  |  |  | 0.06% L. Oil + 507 | 0 | 0 | 2 |
|  |  |  |  | Control + 507 | 0 | 0 | 1 |
| Z.H. | 23 | M | III | 0.04% L. Oil + 0.02% R. Oil + 507 | 0 | 0 | 2 |
|  |  |  |  | 0.06% L. Oil + 507 | 0 | 0 | 2 |
|  |  |  |  | Control + 507 | 0 | 0 | 1 |
| K.B. | 46 | F | III | 0.04% L. Oil + 0.02% R. Oil + 507 | 0 | 0 | 2 |
|  |  |  |  | 0.06% L. Oil + 507 | 0 | 0 | 2 |
|  |  |  |  | Control + 507 | 0 | 0 | 1 |
| O.K. | 29 | M | III | 0.04% L. Oil + 0.02% R. Oil + 507 | 0 | 0 | 2 |
|  |  |  |  | 0.06% L. Oil + 507 | 0 | 0 | 2 |
|  |  |  |  | Control + 507 | 0 | 0 | 2 |
| H.B. | 34 | M | III | 0.04% L. Oil + 0.02% R. Oil + 507 | 0 | 0 | 2 |
|  |  |  |  | 0.06% L. Oil + 507 | 0 | 0 | 2 |
|  |  |  |  | Control + 507 | 0 | 0 | 1 |

## EXAMPLE IV

The same 10 subjects of Example III had the same composition formulations applied to respective test sites and were subjected to a dose of full spectrum solar simulated radiation. This dose was equivalent to 1.5 to 2 hours of noon, summer sunlight at the latitude of Philadelphia, Pennsylvania (5 MED's of UV-B wavelengths and 30J/cm² of UV-A wavelengths). Of the 10 subjects tested, six showed accentuation of tanning by one or both essential oil-containing compositions as compared to the respective control sites. The results are shown in Table V.

## TABLE V

### FULL SPECTRUM (SOLAR SIMULATED RADIATION) EXPOSURE
### TO TEST SITES TREATED WITH "ACTIVE" PRODUCTS CONTAINING A SUNSCREEN (Escalol 507 at 3%)
### AND VEHICLE (CONTROL) VIZ. MINERAL OIL + A SUNSCREEN (507, 3%)

| Subject Initials: | Age: | Sex: | Skin Type: | Test Site: | Erythema (hours) 24 Hours | 48 Hours | Pigmentation (10-14 Days) |
|---|---|---|---|---|---|---|---|
| J.W. | 18 | M | III | 0.04% L. Oil + 0.02% R. Oil + 507 | 2 | 1 | 2 |
| | | | | 0.06% L. Oil + 507 | 2 | 1 | 2 |
| | | | | Control (Vehicle) + 507 | 2 | 1 | 2 |
| D.S. | 25 | M | III | 0.04% L. Oil + 0.02% R. Oil + 507 | 1 | 1 | 1 |
| | | | | 0.06% L. Oil + 507 | 1 | 1 | 2 |
| | | | | Control + 507 | 1 | 1 | 1 |
| M.P. | 20 | M | IV | 0.04% L. Oil + 0.02% R. Oil + 507 | 1 | 0 | 2 |
| | | | | 0.06% L. Oil + 507 | 1 | 0 | 2 |
| | | | | Control + 507 | 1 | 0 | 2 |
| R.M. | 35 | M | III | 0.04% L. Oil + 0.02% R. Oil + 507 | 1 | 0 | 2 |
| | | | | 0.06% L. Oil + 507 | 1 | 0 | 2 |
| | | | | Control + 507 | 1 | 0 | 1 |
| R.G. | 33 | M | III | 0.04% L. Oil +0.02% R. Oil + 507 | 1 | 1 | 2 |
| | | | | 0.06% L. Oil + 507 | 1 | 1 | 2 |
| | | | | Control + 507 | 1 | 1 | 2 |

TABLE V (Continued)

**FULL SPECTRUM (SOLAR SIMULATED RADIATION) EXPOSURE
TO TEST SITES TREATED WITH "ACTIVE" PRODUCTS CONTAINING A SUNSCREEN (Escalol 507 at 3%)
AND VEHICLE (CONTROL) VIZ. MINERAL OIL + A SUNSCREEN (507, 3%)**

| Subject Initials: | Age: | Sex: | Skin Type: | Test Site: | Erythema (hours) 24 Hours | 48 Hours | Pigmentation (10-14 Days) |
|---|---|---|---|---|---|---|---|
| V.M. | 22 | M | III | +507 | | | |
| | | | | 0.04% L. Oil + 0.02% R. Oil | 1 | 0 | 2 |
| | | | | 0.06% L. Oil + 507 | 1 | 0 | 1 |
| | | | | Control + 507 | 1 | 0 | 0 |
| E.H. | 23 | M | III | +507 | | | |
| | | | | 0.04% L. Oil + 0.02% R. Oil | 1 | + | 2 |
| | | | | 0.06% L. Oil + 507 | 1 | + | 2 |
| | | | | Control + 507 | 1 | + | 1 |
| K.B. | 46 | F | III | +507 | | | |
| | | | | 0.04% L. Oil + 0.02% R. Oil | 1 | 1 | 2 |
| | | | | 0.06% L. Oil + 507 | 1 | 1 | 2 |
| | | | | Control + 507 | 1 | 1 | 2 |
| O.K. | 29 | M | III | +507 | | | |
| | | | | 0.04% L. Oil + 0.02% R. Oil | 0 | 0 | 3 |
| | | | | 0.06% L. Oil + 507 | 0 | 0 | 3 |
| | | | | Control + 507 | 0 | 0 | 2 |
| H.B. | 34 | M | III | +507 | | | |
| | | | | 0.04% L. Oil + 0.02% R. Oil | 1 | 1 | 3 |
| | | | | 0.06% L. Oil + 507 | 1 | 1 | 3 |
| | | | | Control + 507 | 1 | 1 | 2 |

## EXAMPLE V

Using the same composition formulation of Example IV, but including 0.5 weight percent fragrance, 13 subjects had respective test sites prepared. One and one-half to two hours later, each subject had a single dose of UV-A wavelengths ($40J/cm^2$) to one test site, and full spectrum solar simulated radiation (SSR) (5 MED's UV-B wavelengths) to a second test site. (The composition formulation for SSR irradiation contained 4 weight pecent Escalol 507).

The results are shown in Table VI for the UV-A wavelength irradiation, and in Table VII for the SSR irradiation. There was accentuation on UV-A wavelength tanning in 10 of the 13 subjects (Table VI), and accentuation of tanning in seven of 12 test sites (Table VII) subjected to SSR exposure (subject C.V. had no participation). Most subjects had a mild erythema (minimal sunburn reaction) 24 to 48 hours after exposure as would be expected with full spectrum irradiation at a dose of 5 MED's. This dose is just above the expected sun protection factor value of the test preparations which is about 4.0.

TABLE VI

RESULTS OF INDOOR TESTING WITH UVA FROM A FILTERED XENON-ARC SOLAR SIMULATOR

UVA DOSE + 40J/cm$^2$
Formulations "A" and "B" in Mineral Oil
Formulation "A" = 0.04% Lime Oil, + 0.02% Rue Oil + 3% Escalol 507 + 0.5% Fragrance
Formulation "B" = 0.06% Lime Oil + 3% Escalol 507 + 0.5% Fragrance

Control Formulation:
3% Escalol 507 + 0.5% Fragrance in Mineral Oil

| Subject Initials: | Age: | Sex: | Skin Type: | Test Site: | Erythema (hours) 24 Hours | 48 Hours | Pigmentation (10-14 Days) |
|---|---|---|---|---|---|---|---|
| P.O'N. | 20 | F | III | Formulation A | 0 | 0 | 3 |
|  |  |  |  | Formulation B | 0 | 0 | 3 |
|  |  |  |  | Control | 0 | 0 | 2 |
| C.F. | 20 | M | III | Formulation A | 0 | - | 3 |
|  |  |  |  | Formulation B | 0 | - | 3 |
|  |  |  |  | Control | 0 | - | 2 |
| R.C. | 23 | M | III | Formulation A | 0 | 0 | 2 |
|  |  |  |  | Formulation B | 0 | 0 | 2 |
|  |  |  |  | Control | 0 | 0 | 2 |
| C.V. | 34 | F | III | Formulation A | 0 | 0 | 2 |
|  |  |  |  | Formulation B | 0 | 0 | 2 |
|  |  |  |  | Control | 0 | 0 | 1 |
| T.N. | 20 | F | III | Formulation A | - | 0 | 3 |
|  |  |  |  | Formulation B | - | 0 | 3 |
|  |  |  |  | Control | - | 0 | 2 |

TABLE VI (continued)

RESULTS OF INDOOR TESTING WITH UVA FROM A FILTERED XENON-ARC SOLAR SIMULATOR

| Subject Initials: | Age: | Sex: | Skin Type: | Test Site: | Erythema (hours) 24 Hours | 48 Hours | Pigmentation (10-14 Days) |
|---|---|---|---|---|---|---|---|
| G.S. | 29 | F | III | Formulation A | 0 | 0 | 2 |
| | | | | Formulation B | 0 | 0 | 2 |
| | | | | Control | 0 | 0 | 2 |
| M.C. | 37 | F | III | Formulation A | 0 | 0 | 2 |
| | | | | Formulation B | 0 | 0 | 2 |
| | | | | Control | 0 | 0 | 1 |
| L.S. | 25 | F | III | Formulation A | - | 0 | 1 |
| | | | | Formulation B | - | 0 | 2 |
| | | | | Control | - | 0 | 1 |
| M.P. | 20 | M | IV | Formulation A | 0 | 0 | 3 |
| | | | | Formulation B | 0 | 0 | 3 |
| | | | | Control | 0 | 0 | 1 |
| K.B. | 46 | F | III | Formulation A | 0 | 0 | 2 |
| | | | | Formulation B | 0 | 0 | 2 |
| | | | | Control | 0 | 0 | 2 |
| L.F. | 19 | M | III | --- | - | - | - |
| | | | | Formulation B | 0 | 0 | 3 |
| | | | | Control | 0 | 0 | 1 |
| T.P. | 18 | F | III | --- | - | - | - |
| | | | | Formulation B | 0 | 0 | 2 |
| | | | | Control | 0 | 0 | 1 |
| K.U. | 20 | M | III | --- | - | - | - |
| | | | | Formulation B | 0 | 0 | 2 |
| | | | | Control | 0 | 0 | 1 |

0 257 928

## TABLE VII

### RESULTS OF INDOOR TESTING WITH FULL SPECTRUM SOLAR SIMULATED RADIATION

DOSE = 5 MED's + 35J/cm$^2$ UVA
Formulations "A" and "B" in Mineral Oil
Formulation "A" = 0.04% Lime Oil, + 0.02% Rue Oil + 4% Escalol 507 + 0.5% Fragrance
Formulation "B" = 0.06% Lime Oil + 3% Escalol 507 + 0.5% Fragrance

Control Formulation:
4% Escalol 507 + 0.5% Fragrance in Mineral Oil

| Subject Initials: | Age: | Sex: | Skin Type: | Test Site: | Erythema (hours) 24 Hours | 48 Hours | Pigmentation (10-14 Days) |
|---|---|---|---|---|---|---|---|
| P.O'N | 20 | F | III | Formulation A | 1 | 1 | 2 |
| | | | | Formulation B | 1 | 1 | 2 |
| | | | | Control | 1 | 1 | 2 |
| C.F. | 20 | M | III | Formulation A | 1 | - | 2 |
| | | | | Formulation B | 1 | - | 2 |
| | | | | Control | 1 | - | 1 |
| R.C. | 23 | M | III | Formulation A | 1 | 1 | 2 |
| | | | | Formulation B | 1 | 1 | 2 |
| | | | | Control | 1 | 1 | 1 |
| C.V. | 34 | F | III | Formulation A | - | - | - |
| | | | | Formulation B | - | - | - |
| | | | | Control | - | - | - |
| T.N. | 20 | F | III | Formulation A | - | 0 | 1 |
| | | | | Formulation B | - | 0 | 1 |
| | | | | Control | - | 0 | 1 |
| G.S. | 29 | F | III | Formulation A | 0 | 0 | 1 |
| | | | | Formulation B | 0 | 0 | 1 |
| | | | | Control | 0 | 0 | 0 |

TABLE VII (Continued)

RESULTS OF INDOOR TESTING WITH FULL SPECTRUM SOLAR SIMULATED RADIATION

| Subject Initials: | Age: | Sex: | Skin Type: | Test Site: | Erythema (hours) 24 Hours | 48 Hours | Pigmentation (10-14 Days) |
|---|---|---|---|---|---|---|---|
| M.C. | 37 | F | III | Formulation A | 1 | 0 | 3 |
| | | | | Formulation B | 1 | 0 | 3 |
| | | | | Control | 1 | 0 | 3 |
| L.S. | 25 | F | III | Formulation A | - | 1 | 2 |
| | | | | Formulation B | - | 1 | 2 |
| | | | | Control | - | 1 | 2 |
| M.P. | 20 | M | IV | Formulation A | 1 | 0 | 2 |
| | | | | Formulation B | 1 | 0 | 3 |
| | | | | Control | 1 | 0 | 1 |
| K.B. | 46 | F | III | Formulation A | 2 | 1 | 2 |
| | | | | Formulation B | 2 | 1 | 2 |
| | | | | Control | 2 | 1 | 1 |
| L.F. | 19 | M | III | ---- | - | - | - |
| | | | | Formulation B | 1 | 0 | 1 |
| | | | | Control | 1 | 0 | 2 |
| T.P. | 18 | F | III | ---- | - | - | - |
| | | | | Formulation B | 1 | 1 | 2 |
| | | | | Control | 1 | 1 | 1 |
| K.U. | 20 | M | III | ---- | - | - | - |
| | | | | Formulation B | 1 | 1 | 2 |
| | | | | Control | 1 | 1 | 1 |

0 257 928

17

## EXAMPLE VI

Using the same composition formulations of Example V with the 3 weight percent Escalol 507 present, 14 subjects had large test sites (approximately 20 cm x 30 cm) prepared on their respective backs. The formulations were rubbed in very well one hour (11:00 a.m.) prior to natural sunlight exposure, and applied again immediately before sunlight exposure by massaging into the skin. The test sites were exposed to sunlight from 12:00 noon to 2:30 p.m. on sunny, cloudless days. Erythema was graded 24 hours later, while pigmentation was graded 10-14 days later. Seven subjects' composition included lime oil (0.04 weight percent) plus rue oil (0.02 weight percent), and seven subjects' composition included lime oil (0.06 weight percent). All controls were identical.

The results are shown in Table VIII. Aside from minimal sunburn erythema which was present 24 hours alter, both sites (treated and control) for all subjects developed a uniform, cosmetically acceptable tan 10-14 days later. Both essential oil-containing produced enhancement of tanning in four of each seven tested. The differences in tanning, where present, were mild and of a one-point grade. No untoward reactions (e.g. phototoxicity, streaking, lack of tanning uniformity, etc.) were observed in any of the subjects.

## TABLE VIII

RESULTS OF SUNEXPOSURE WITH TWO TEST FORMULATIONS (A & B).  ALL EXPOSURES
GIVEN FROM 12 Noon to 2:30 P.M. ON CLOUDLESS SUNNY DAYS (May 15 to June 15, 1986)

Formulations "A" and "B" in Mineral Oil
Formulation "A" = 0.04% Lime Oil + 0.02% Rue Oil + 3% Escalol 507 + 0.5% Fragrance
Formulation "B" = 0.06% Lime Oil + 3.0% Escalol 507 + 0.5% Fragrance

Control Formulation:
3% Escalol 507 + 0.5% Fragrance in Mineral Oil

| Subject Initials: | Age: | Sex: | Skin Type: | Test Site: | Erythema (hours) 24 Hours | Pigmentation (10-14 Days) |
|---|---|---|---|---|---|---|
| M.P. | 20 | M | IV | Formulation A | 0 | 2 |
| | | | | Control | 0 | 1 |
| C.M. | 23 | F | III | Formulation A | 0 | 1 |
| | | | | Control | 0 | 1 |
| C.V. | 34 | F | III | Formulation A | 1+ | 2 |
| | | | | Control | 1+ | 2 |
| S.L. | 21 | F | III | Formulation A | 1+ | 2 |
| | | | | Control | 1+ | 1 |
| J.K. | 20 | F | III | Formulation A | 1+ | 2 |
| | | | | Control | 1+ | 1 |
| R.C. | 23 | M | III | Formulation A | 1+ | 1 |
| | | | | Control | 1+ | 2 |
| T.P. | 18 | M | III | Formulation A | 1+ | 2 |
| | | | | Control | 1+ | 1 |

## TABLE VIII (Continued)

RESULTS OF SUNEXPOSURE WITH TWO TEST FORMULATIONS (A & B). ALL EXPOSURES GIVEN FROM 12 Noon to 2:30 P.M. ON CLOUDLESS SUNNY DAYS (May 15 to June 15, 1986)

| Subject Initials: | Age: | Sex: | Skin Type: | Test Site: | Erythema (hours) 24 Hours | Pigmentation (10-14 Days) |
|---|---|---|---|---|---|---|
| A.McG. | 31 | F | III | Formulation B | 1+ | 2 |
|  |  |  |  | Control | 1+ | 2 |
| L.W. | 42 | F | III | Formulation B | + | 2 |
|  |  |  |  | Control | ++ | 1 |
| B.M. | 34 | F | III | Formulation B | 1+ | 2 |
|  |  |  |  | Control | 1+ | 1 |
| D.S. | 25 | M | III | Formulation B | 1+ | 2 |
|  |  |  |  | Control | 1+ | 1 |
| T.N. | 20 | F | III | Formulation B | 1+ | 2 |
|  |  |  |  | Control | 1+ | 1 |
| H.B. | 34 | M | III | Formulation B | 0 | 2 |
|  |  |  |  | Control | 0 | 2 |
| B.S. | 22 | M | III | Formulation B | 2+ | 2 |
|  |  |  |  | Control | 2+ | 2 |

## Toxocologic Evaluations

Two compositions were prepared. Composition A contained 0.04 weight percent Lime Oil, 0.02 weight percent Rue Oil, 3.0 weight pecent Escalol 507, and a creamy lotion carrier vehicle. Composition B contained 0.06 weight pecent Lime Oil, 3.0 weight percent Escalol 507, and a creamy lotion carrier vehicle.

Compositions A and B were both tested in 25 subjects in a routine photomaximization assay for photocontact allergenicity. No adverse reaction of any kind and no instances of photocontact allergy were observed.

Compositions A and B were subjected to a routine maximization assay in 25 subjects. Separate panels of 25 subjects each were used for each composition. No adverse or untoward reactions of any kind were observed, and no instances of contact hypersensitivity developed.

Compositions A and B were examined for primary irritancy potential in a routine 10-day primary irritation test in 10 subjects. No significant irritation was observed with either composition.

Finally, compositions A and B were both assayed in vivo for phototoxicity potential in 10 healthy volunteers. No instances of phototoxicity were observed with either composition.

It is to be understood that the above-described preferred embodimetns are illustrative and are not meant to be limiting, and that the scope of the instant invention is defined by the claims which now follow.

## Claims

1. A suntanning composition for topical application to skin to enhance melanogenesis caused by ultraviolet-A wavelengths, said composition comprising a carrier vehicle and at least one effective essential oil in an amount which promotes said melanogenesis in the presence of ultraviolet-A wavelengths.

2. A suntanning composition as claimed in Claim 1 wherein the essential oil is rue oil.

3. A suntanning composition as claimed in Claim 2 wherein the rue oil is present in an amount from about 0.01 weight percent to about 0.5 weight percent of the entire composition.

4. A suntanning composition as claimed in Claim 3 wherein the rue oil is present in an amount from about 0.02 weight percent to about 0.1 weight percent of the entire composition.

5. A suntanning composition as claimed in Claim 4 wherein the rue oil is present in an amount from about 0.06 weight percent to about 0.1 weight percent of the entire composition.

6. A suntanning composition as claimed in Claim 2 comprising in addition lime oil as an essential oil.

7. A suntanning composition as claimed in Claim 6 wherein the total amount of rue oil and lime oil present is from about 0.01 weight percent to about 1.0 weight percent of the entire composition.

8. A suntanning composition as claimed in Claim 7 wherein the rue oil is present in an amount of about 0.02 weight percent of the entire composition and the lime oil is present in an amount of about 0.04 weight percent of the entire composition.

9. A suntanning composition as claimed in Claim 1 wherein the essential oil is a citrus oil.

10. A suntanning composition as claimed in Claim 9 wherein the citrus oil is lime oil.

11. A suntanning composition as claimed in Claim 10 wherein the lime oil is present in an amount from about 0.01 weight percent to about 1.0 weight percent of the entire composition.

12. A suntanning composition as claimed in Claim 11 wherein the lime oil is present in an amount from about 0.02 weight percent to about 0.1 weight percent of the entire composition.

13. A suntanning composition as claimed in Claim 12 wherein the lime oil is present in an amount from about 0.06 weight percent to about 0.1 weight percent of the entire composition.

14. A suntanning composition as claimed in any one of the preceding claims comprising in addition an ultraviolet-B wavelength screen.

15. A suntanning composition as claimed in Claim 14 wherein the ultraviolet-B wavelength screen is present in an amount from about 1 weight percent to about 7 weight percent of the entire composition.

16. A suntanning composition as claimed in Claim 15 wherein the ultraviolet-B wavelength screen is present in an amount of about 3 weight percent of the entire composition.

17. A suntanning composition as claimed in Claim 15 wherein the essential oil is rue oil present in an amount of about 0.06 weight percent to about 0.01 weight percent of the entire composition.

18. A suntanning composition as claimed in Claim 15 wherein the essential oil is lime oil present in an amount from about 0.01 weight percent to about 1.0 weight percent of the entire composition.

19. A suntanning composition as claimed in Claim 15 comprising rue oil in an amount of about 0.02 weight percent of the entire composition and lime oil in an amount of about 0.04 weight pecent of the entire composition.

20. A method of acquiring a tan in the presence of ultraviolet-A wavelengths, said method comprising:

a. topically applying to skin an effective amount of a suntanning composition according to any one of the preceding claims, and

b. exposing said skin to ultraviolet-A wavelengths for a time period adequate to produce skin tanning.